(19) Europäisches Patentamt
European Patent Office
Office européen des brevets

(11) **EP 1 700 614 B1**

(12) **EUROPÄISCHE PATENTSCHRIFT**

(45) Veröffentlichungstag und Bekanntmachung des Hinweises auf die Patenterteilung:
**08.05.2013 Patentblatt 2013/19**

(51) Int Cl.:
***A61M 11/06*** *(2006.01)* ***A61M 16/00*** *(2006.01)*

(21) Anmeldenummer: **05005033.5**

(22) Anmeldetag: **08.03.2005**

(54) **Inhalationsvorrichtung**

Inhalation device

Dispositif d'inhalation

(84) Benannte Vertragsstaaten:
**AT BE BG CH CY CZ DE DK EE ES FI FR GB GR HU IE IS IT LI LT LU MC NL PL PT RO SE SI SK TR**

(43) Veröffentlichungstag der Anmeldung:
**13.09.2006 Patentblatt 2006/37**

(73) Patentinhaber: **Activaero GmbH**
**35285 Gemünden (DE)**

(72) Erfinder:
• **Müllinger, Bernhard**
**80634 München (DE)**
• **Wenker, Andreas**
**86899 Landsberg am Lech (DE)**
• **Körber, Dorothee**
**86157 Augsburg (DE)**
• **Scheuch, Gerhard, Dr.**
**35288 Wohratal (DE)**

(74) Vertreter: **Vossius & Partner**
**Siebertstrasse 4**
**81675 München (DE)**

(56) Entgegenhaltungen:
**EP-A- 1 258 264      EP-A- 1 491 227**
**WO-A-00/58022      WO-A-98/52633**
**WO-A2-01/32069     WO-A2-2005/016217**
**DE-A1- 10 022 795    DE-A1- 19 939 417**
**DE-U1-202004 004 809   US-A1- 2001 054 421**
**US-B1- 6 644 310**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft eine Vorrichtung zur Applikation eines Medikaments über die Lunge.

Die Verwendung des Inhalationsweges für die Applikation von Medikamenten gewinnt zunehmend an Bedeutung. Dabei werden außer dem Einsatz neuer lokal wirkender Medikamente für die Therapie von Lungenerkrankungen auch neue Therapiestrategien entwickelt, die die Lunge als Eingangsorgan für systemisch wirksame Substanzen verwenden.

Bei der Applikation von Medikamenten mittels Inhalation werden wachsende Anforderungen an die Qualität des Inhalationsvorganges gestellt. Die vom Arzt verordnete Medikamentendosis muß möglichst genau und reproduzierbar appliziert werden, wobei die verabreichte Dosis und ihre Reproduzierbarkeit im wesentlichen von dem Atemmanöver abhängt.

Im klinischen Alltag läßt sich das vom Patienten verwendete Atemmanöver nur wenig beeinflussen und entzieht sich der ärztlichen Kontrolle, so dass eine exakte Vorgabe von Atemvolumen und Atemfluss bei der Inhalation therapeutischer Aerosole wünschenswert ist. Damit wäre eine erhebliche Verbesserung der Inhalationstherapie und ihrer Reproduzierbarkeit verbunden.

Die Verabreichung von Arzneistoffen in Aerosolform durch Inhalation in die Lunge ist im wesentlichen durch vier Faktoren beeinflusst: (i) die Partikelgröße und die Partikeleigenschaften des Aerosols; (ii) das Atemzugvolumen des Patienten; (iii) den Atemfluss des Patienten; und (iv) die Morphometrie und Atemwege des Patienten. Während bisherige Systeme zwar Aerosole in geeigneten Teilchengrößenbereichen produzieren, werden bei bekannten Systemen die Parameter "Atemzugvolumen" und "Atemfluss" (Atemgeschwindigkeit) nur ungenügend oder gar nicht berücksichtigt. Dies führt zu einer unkontrollierten Inhalation des Aerosols, was wiederum dazu führt, dass eine unzureichende Menge an Aerosolteilchen die Lunge erreicht bzw. innerhalb der Lunge die zu therapierenden Bereiche (beispielsweise Alveolarbereich) nicht erreicht.

Aus der WO 98/52633 ist eine Vorrichtung zur Applikation eines Medikaments über die Lunge bekannt, die aus einem Inhalationsmundstück mit einem zugeordneten einstellbaren Vernebler und aus einem Druckluftsteuerventil besteht, durch das ein voreinstellbarer Volumenstrom von Druckluft während einer einstellbaren Zeitdauer an den das flüssige Medikament enthaltenden Vernebler lieferbar ist. Ein solches Druckluftsteuerventil ist bei der bekannten Inhalationsvorrichtung unabdingbar, da hohe Drücke notwendig sind, um das Medikament zu zerstäuben.

Die EP-A-1 258 264 offenbart eine Vorrichtung zum Verabreichen von Aerosolen, bei der Inhalationsfluß und Inhalationsvolumen abhängig von individuellen Patienten- und/oder Aerosolparametern eingestellt werden.

WO 00/58022 beschreibt ein Verfahren und eine Vorrichtung zum Vernebeln einer Flüssigkeit zur Verabreichung eines Aerosols. Bei dem beschriebenen Verfahren wird eine zu zerstäubende Flüssigkeit in ein Reservoir eingegeben, in dem es mit der Oberfläche eines starren porösen Körpers in Kontakt kommt. Eine andere Fläche dieses Körpers wird dann mit Druckluft beaufschlagt und der Körper mit Ultraschallfrequenz bewegt.

DE-A-199 39 417 beschreibt eine Inhalationsvorrichtung mit einem mit Druckluft betriebenen Vernebler und betrifft insbesondere die Steuerung des Verneblers.

DE-A-100 22 795 beschreibt ein atemgesteuertes Inhalationstherapiegerät, bei dem mittels eines Infrarotsensors die Aerosolwolke gemessen und dementsprechend der Vernebler aktiviert wird.

DE 20 2004 004 809 U1 beschreibt ein Atemtherapiegerät, das mit einem Kompressor Luftimpulse erzeugt.

Der Erfindung liegt die Aufgabe zugrunde, eine funktionell einfacher konzipierte Vorrichtung bereitzustellen. Diese Aufgabe wird durch die im Patentanspruch 1 genannten Merkmale gelöst. Bevorzugte Merkmale, die die Erfindung vorteilhaft weiterbilden, sind den nachgeordneten Patentansprüchen zu entnehmen.

Die Erfindung geht von dem Grundgedanken aus, ein gewünschtes bzw. vorgegebenes Inhalationsvolumen bzw. einen gewünschten bzw. vorgegebenen Inhalationsfluss durch eine entsprechende Ansteuerung der Luftpumpe bereitzustellen. Dies ist mit dem Vorteil verbunden, dass zur Drosselung bzw. Steuerung des Inhalationsvolumens/Inhalationsflusses keine Ventile, mit Ausnahme eines Überdruckventils als Sicherheitsventil, notwendig sind. Bei bekannten Inhalationsvorrichtungen sind zwingend Drosselventile notwendig, um die zur Zerstäubung des Medikaments notwendigen hohen Drücke handhaben zu können.

Zur Steuerung der Luftpumpe ist bei der erfindungsgemäßen Inhalationsvorrichtung eine Steuereinrichtung vorgesehen, die die Luftpumpe derart ansteuert, dass sie einen Inhalationsfluss und/oder ein Inhalationsvolumen gemäß einem vorgegebenen zeitlichen Verlauf an einen an die Luftpumpe angeschlossenen Vernebler liefert. Der Vernebler weist ein Mundstück auf, über das der Patient inhaliert.

Der Inhalationsfluss und/oder das Inhalationsvolumen ist während des vorgegebenen zeitlichen Verlaufs im wesentlichen konstant.

Unter vorgegebenem Inhalationsfluss bzw. vorgegebenem Inhalationsvolumen wird bevorzugt ein Inhalationsfluss bzw. Inhalationsvolumen verstanden, das abhängig von Patientenparametern und/oder Aerosolparametern für eine konkrete Behandlung eines konkreten Patienten, die für diesen Patienten vor der Inhalation ermittelt werden, mit dem eine optimale Inhalation bzw. Behandlung des Patienten erreicht wird. Unter optimaler Inhalation ist dabei eine

EP 1 700 614 B1

gezielte individuelle Deposition der zu verabreichenden individuellen Wirkstoffdosis in die Lunge oder gezielt ausgewählte Bereiche der Lunge zu verstehen, also die gezielte Deposition einer vorherbestimmten Wirkstoffmenge in einen vorherbestimmten Bereich der Lunge. Die Individualisierung erfolgt hierbei abhängig von den individuellen Patienten- und/oder Aerosolparametern, d.h. abhängig von mindestens einem dieser Parameter wird vorausberechnet, welche Wirkstoffdosis zur Behandlung bei Deposition in den gewünschten Bereich der Lunge notwendig ist.

Die Steuerung der Luftpumpe mittels der Steuereinrichtung erfolgt über die Spannung oder Pulsweitenmodulation eines Rechtecksignals mit konstanter bzw. variabler Spannung. Dies bedeutet, dass die Steuerspannung der Luftpumpe von der Steuereinrichtung derart vorgegeben bzw. geregelt wird, dass die Luftpumpe den gewünschten bzw. vorgegebenen Inhalationsfluss bzw. das gewünschte bzw. vorgegebene Inhalationsvolumen erzeugt. Erfindungsgemäß wird also über eine direkte Ansteuerung der Luftpumpe der gewünschte Inhalationsfluss bzw. das gewünschte Inhalationsvolumen eingestellt (und nicht wie bisher über zusätzliche Drosselventile). Dies erfolgt vorzugsweise abhängig von Patienten-individuellen Lungenfunktionsparametern, wie oben erläutert. Diese Patienten-individuellen Lungenfunktionsparameter sind vorzugsweise Inhalationsfluss und/oder Inhalationsvolumen des individuellen Patienten, also das Lungenvolumen des Patienten (gegebenenfalls mit einem Sicherheitsabschlag) und der vom Patienten erzielbare oder als angenehm empfundene Inhalationsfluss. Gemäß einer bevorzugten erfindungsgemäßen Ausführungsform sind von diesen patienten-individuellen Lungenfunktionsparameter abgeleitete bzw. berechnete Patientendaten, die diesen Lungenfunktionsparametern entsprechen, auf einem Speichermedium hinterlegt, das von der Inhalationsvorrichtung mit einer geeigneten Leseeinrichtung lesbar ist. Beispielsweise werden diese Daten von dem behandelnden Arzt oder der betreuenden Institution auf dem Speichermedium gespeichert. Das Speichermedium ist entweder ein internes Speichermedium, also ein Speichermedium der Inhalationsvorrichtung, auf das zugreifbar ist (z.B. über Tasteneingaben oder Datenleitungen und Modeme), oder ein separates, externes Speichermedium. Beispielsweise ist der Datenträger eine SmartCard, und die Leseeinrichtung ein SmartCard-Leser. Auf der SmartCard werden die Patienten-individuellen Lungenfunktionsparameter bzw. die entsprechenden Patientendaten beispielsweise von dem behandelnden Arzt hinterlegt bzw. gespeichert. Die SmartCard enthält alle für die Inhalation notwendigen individuellen Daten, so dass der Patient beispielsweise auch zu Hause mit der erfindungsgemäßen Inhalationsvorrichtung das notwendige Medikament inhalieren kann, also dies nicht unter Aufsicht des behandelnden Arztes tun muß.

Die Steuereinrichtung berechnet auf der Grundlage der auf dem Speichermedium abgelegten Patienten-individuellen Daten entsprechende Steuerparameter, aus denen auf die Pumpenspannung geschlossen werden kann, die zur Erzielung des notwendigen Inhalationsflusses notwendig ist bzw. eingestellt werden muß.

Vorzugsweise wird für jede Inhalationsvorrichtung die Kennlinie der darin verwendeten Luftpumpen zuerst individuell messtechnisch ermittelt und anschließend mathematisch angenähert. Diese mathematische Annäherung wird dann in der Steuereinrichtung für den laufenden Betrieb der Inhalationsvorrichtung zur Durchführung der Korrektur hinterlegt. Vorzugsweise erfolgt dies erfindungsgemäß mittels folgender Formel:

$$U = x\frac{V}{l\,/\,\min}\left(Q_{Soll} - Q_{Messung}\right) + U\left(Q_{Messung}\right) - yV \cdot e^{\frac{z}{Zyklenzahl}}$$

wobei:

$Q_{Soll}$:　　　gewünschter Fluss in l/min
$Q_{Messung}$:　　gemessener Fluss in l/min (am Anfang) bei der Spannung $U(Q_{Messung})$
x, y, z:　　　aus Messungen ermittelte Konstanten.

Bevorzugte Werte für die Parameter x und y liegen in den Bereichen von 0,3 < x < 0,9 bzw. 0,6 < y < 1,2. Mehr bevorzugt ist x = 0,59 und y = 0,89. Bevorzugte Werte für z liegen im Bereich von 3000< z < 5000. Mehr bevorzugt ist z=4000.

In der oben angegebenen bevorzugten Formel für die Ermittlung der Steuerspannung wird für eine konkret verwendete Luftpumpe der Verlauf des Flusses abhängig von der Zyklenzahl berücksichtigt. Diese Kennlinie Fluss vs. Zyklenzahl ändert sich von Luftpumpe zu Luftpumpe, und ferner ist der Fluss den eine Luftpumpe bereitstellt, über die Zyklenzahl nicht konstant.

Zur Überwachung des Inhalationsflusses bzw. Inhalationsvolumens während der Inhalation ist vorzugsweise ein Drucksensor vorgesehen. Durch das von diesem Drucksensor bereitgestellte Signal kann die Steuereinrichtung den tatsächlichen Inhalationsfluss (Ist-Fluss) mit dem vorgegebenen Inhalationsfluss (Soll-Fluss) vergleichen und daraufhin gegebenenfalls die Steuerspannung der Luftpumpe variieren bzw. regeln, um Abweichungen des tatsächlichen Inhalationsflusses von dem vorgegebenen Inhalationsfluss zu beseitigen. Vorzugsweise erfolgt dies erfindungsgemäß mittels folgender Formel:

$$\Delta p = xQ^2 + yQ$$

wobei:

Δp:  Druckdifferenz
Q:  Fluss
x, y:  aus Messungen ermittelte Konstanten

Der Drucksensor wird bevorzugt auch zur Fehlerermittlung verwendet. Wenn der vom Drucksensor gemessene Druck vom Druck der Pumpe abweicht, kann dies beispielsweise auf einen zugesetzten Filter hindeuten, der ausgewechselt werden muss.

Ferner bevorzugt berücksichtigt die Steuereinrichtung den vom Patienten während der Inhalation bewußt oder unbewußt erzeugten Gegendruck. Dies erfolgt beispielsweise über entsprechende Kennlinien, die in der Steuereinrichtung hinterlegt sind. Abhängig vom Gegendruck des Patienten wird für eine bestimmte Steuerspannung der Luftpumpe ein Inhalationsfluss von der Luftpumpe erzeugt. Wenn der Gegendruck des Patienten steigt, nimmt der Inhalationsfluss für diese bestimmte Spannung ab. Entsprechend nimmt der Inhalationsfluss für eine feste Spannung zu, wenn der Gegendruck sinkt. Um derartige Schwankungen bzw. Beeinflussungen durch den Patienten auszugleichen, wird abhängig vom Gegendruck des Patienten die Steuerspannung der Luftpumpe so variiert bzw. nachgeregelt, dass der Fluss im wesentlichen konstant bleibt.

Die Steuereinrichtung der erfindungsgemäßen Inhalationsvorrichtung weist vorzugsweise eine Steuereinheit, also eine Motorsteuerung für den Antrieb der Luftpumpe auf, eine Verarbeitungseinheit in Form eines Mikroprozessors, sowie einen Speicher (Arbeitsspeicher). Ferner weist die Inhalationsvorrichtung vorzugsweise eine Anzeigeeinheit und eine Eingabeeinheit auf.

Das schon erwähnte Überdruckventil als auch der erwähnte Drucksensor befinden sich in beliebiger Reihenfolge abstromseitig von der Luftpumpe.

Die Luftpumpe der Inhalationsvorrichtung ist ein Kompressor.

Ein weiterer Aspekt der vorliegenden Erfindung betrifft die Steuerung des Inhalationsvorganges. Gemäß diesem Aspekt der vorliegenden Erfindung wird dem Patienten die Möglichkeit bereitgestellt, das zu inhalierende Medikament zu wählen, wenn der Patient mit mehreren Medikamenten behandelt wird. Ferner wird dem Patienten die Möglichkeit gegeben, das Inhalationsvolumen individuell zu modifizieren und von dem vorgegebenen Inhalationsfluss abzuweichen, beispielsweise abhängig von der jeweiligen "Tagesform". Sobald dann der Patient mit der Inhalation beginnt und der Drucksensor dies erkennt (ein Saugen des Patienten am Mundstück wird vom Drucksensor als Start der Inhalation erkannt), betrachtet die Steuereinrichtung die unmittelbar zuvor getroffene Auswahl des Patienten als fest eingestellt. Wird die Inhalation erfolgreich zu Ende geführt, bleiben die vom Patienten vorgegebenen, d.h. modifizierten Parameter in der Steuereinrichtung gespeichert und der nächste Inhalationsvorgang beginnt mit dem vor der vorherigen Inhalation ausgewählten "alten" Inhalationsvolumen bzw. Inhalationsfluss und dem vor der vorherigen Inhalation ausgewählten Medikament, sofern der Patient keine Neueinstellung vornimmt. Wird jedoch der Inhalationsvorgang vom Patienten abgebrochen, weil beispielsweise das ausgewählte bzw. modifizierte Inhalationsvolumen für den Patienten nicht angenehm ist, fragt die Steuereinrichtung nach, ob die begonnene Inhalation fortgesetzt werden oder abgebrochen werden soll. Im letzteren Falle fordert die Steuereinrichtung den Patienten zur Neueinstellung der Parameter auf.

Erfindungsgemäß erfolgt die Erkennung des Beginns des Inhalationsvorganges über den Drucksensor. Sobald die Steuereinrichtung den Beginn eines Inhalationsvorganges erkennt, wird die Luftpumpe aktiviert. Mit anderen Worten, die Luftpumpe ist erfindungsgemäß nicht kontinuierlich in Betrieb, sondern lediglich während des Inhalationsvorganges bzw. Inspirationsvorganges, nicht jedoch während den Pausen und Exspirationsvorgängen. Die erfindungsgemäße Inhalationsvorrichtung dieses Aspekts weist eine Luftpumpe und einen Drucksensor zum Erfassen des Beginns eines Inhalationsvorganges auf, wobei das zu inhalierende Medikament und/oder der Inhalationsfluss und/oder das Inhalationsvolumen vom Nutzer individuell einstellbar ist, und wobei bei Erfassung der ersten Inhalationsvorganges durch den Drucksensor der/die individuell eingestellten Parameter fest eingestellt werden und die Luftpumpe aktiviert wird

Durch die erfindungsgemäße Vorrichtung wird es dem Patienten daher mit einfachen Mitteln ermöglicht, ein exakt vorgegebenes Pharmakonvolumen mit exakt vorgegebenem Atemfluss zu inhalieren. Der Patient wird quasi beatmet, da die Inhalationsvorrichtung den Atemfluss vorgibt und einstellt. Dabei wird in günstiger Weise die im klinischen Alltag für Therapie-Vernebler bereits vorgesehene separate Luftzuführung derart gesteuert, dass nur ein vorgegebenes Luftvolumen mit einem vorgegebenen Fluss an den Vernebler abgegeben wird. Nach der Erfindung wird somit die Luft des Vernebler auf eine vorwählbare Volumenstromstärke eingestellt, die vom Patienten nicht beeinflusst werden kann, wodurch sich eine Flusskonstanz ergibt. Der Zuluftvolumenstrom wird dann bevorzugt von einer elektronischen Steuerungseinheit über einen fest vorgegebenen Zeitraum eingeschaltet, um so ein exakt vorgegebenes Luftvolumen zu applizieren.

Gemäß einer bevorzugten Ausgestaltung ist vor dem einstellbaren Vernebler ein Regler für das Konstanthalten des Volumenstroms angeordnet, wobei bevorzugt zur Kontrolle eines vorbestimmten Volumenstroms dem Regler ein Druckmesser nachgeordnet ist.

Für den praktischen Gebrauch der Vorrichtung ist es weiterhin günstig, wenn zur Triggerung des Verneblungsbeginns des Verneblers ein vorzugsweise auf Saugdruck im Inhalationsmundstück ansprechender Druckmesser vorgesehen ist.

Vorteilhaft kann gemäß der Erfindung durch die gleichzeitige Wahl von Inhalationsfluss und inhaliertem Volumen ein breites Spektrum von Atemmanövern der medizinischen Anwendung erschlossen werden, wobei über unterschiedliche Atemmanöver der Depositionsort in der Lunge in gewünschter Weise beeinflussbar ist. Damit ist es möglich, Medikamente, die beispielsweise in zentralen Lungenbereichen wirken sollen, überwiegend auch dort zu deponieren.

Ein weiterer Vorteil der erfindungsgemäßen Vorrichtung besteht darin, dass damit eine routinemäßige Durchführung von Inhalationen bei Patienten im klinischen Bereich verbessert werden kann, weil ein Patient durch die vorgesehene Atemtriggerung der Inhalation keine Probleme hinsichtlich der Synchronisierung des Starts der Medikamentenverneblung und des Beginns der Inhalation hat. Außerdem können vorteilhaft Fehler bei der Inhalation, die auf eine unsachgemäße Handhabung des Verneblers zurückzuführen sind, deutlich vermindert werden.

Nachfolgend wird die Erfindung unter Bezugnahme auf die beigefügten Zeichnungen näher erläutert. Es zeigen:

Fig. 1     eine schematisierte Darstellung einer Ausführungsform einer erfindungsgemäßen Vorrichtung;
Fig. 2     eine Darstellung des Flusses in Abhängigkeit von Spannung der Pumpe und des Gegendruckes des Patienten;
Fig. 3     die Veränderung des von einer Pumpe bereitgestellten Flusses abhängig von der Zykluszahl; und
Fig. 4     eine bevorzugte Lagerung der Luftpumpe in der Inhalationsvorrichtung.

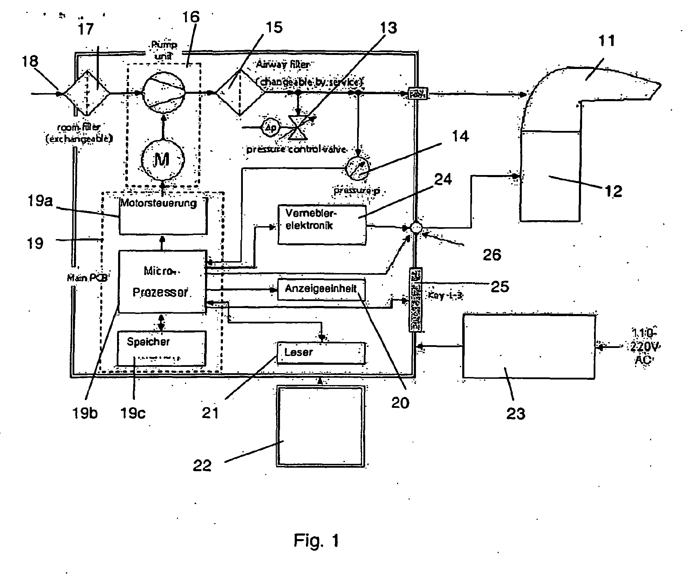

In Figur 1 ist schematisiert das Zusammenwirken einzelner Komponenten einer erfindungsgemäßen Inhalationsvorrichtung 10 dargestellt. Die Vorrichtung 10 zur Applikation eines Medikament-Aerosols über die Lunge besteht aus einem Inhalationsmundstück 11 mit einem zugeordneten Vernebler 12. In Leitungsverbindung mit dem Inhalationsmundstück 11 befinden sich ein Überdruckventil 13, ein Drucksensor 14, ein austauschbares Filter 15 (oder Druckdämpfer), eine (Druck-)Luftpumpe 16, ein weiteres austauschbares Filter 17, und ein Lufteingang 18. Der Filter 17 ist dem Lufteingang 18 zugeordnet, um die Zuluft zu der Luftpumpe 16 zu filtern. Filter 15, Drucksensor 14 und Überdruckventil 13 sind abstromseitig von der Luftpumpe 16 vorgesehen. Der Drucksensor 14 spricht zur Triggerung des Verneblungsbeginns des Verneblers 12 auf Saugdruck im Mundstück 11 an.

Eine elektronische Steuerungseinheit 19 mit einer Motorsteuerung 19a, einem Mikroprozessor 19b und einem Speicher 19c ist funktionell mit der Pumpe 16, dem Drucksensor 14 und über entsprechende Verneblerelektronik 24 mit dem Vernebler 12 verbunden. Die Verneblerelektronik ist vorzugsweise einfach austauschbar, um so für den jeweils verwendeten Vernebler die zugehörige Elektronik verwenden zu können. Der Vernebler ist über einen Anschlusspunkt 26 mit der Inhalationsvorrichtung verbindbar. Vorzugsweise ist der Anschluss 26 ein kodierter Anschluss, der somit eine automatische Erkennung des angeschlossenen Verneblers ermöglicht. Dies erlaubt, eine Plausibilitätsprüfung durchzuführen, beispielsweise derart, ob das zu verabreichende Medikament überhaupt mit dem angeschlossenen Vernebler appliziert werden kann.

Ferner ist eine optische Anzeige 20 vorgesehen, um dem Patienten den aktuellen Inhalationsfluss anzuzeigen. Die Anzeige dient auch zur Anzeige des Namens des gerade verwendeten Medikamentes, Fehlermeldungen, etc. Die Anzeige dient vorzugsweise auch dazu, Fehlermeldungen des Verneblers anzuzeigen. So kann beispielsweise bei Verwendung eines Flüssigverneblers für den Fall, dass sich keine Flüssigkeit mehr im Vernebler befindet, eine Aufforderung zum Nachfüllen, Abbrechen oder eine Defektanzeige angezeigt werden.

Der Drucksensor 14 dient zum Konstanthalten des Inhalationsflusses in einem Wertebereich von beispielsweise 0 - 1000 $cm^3$/s.

Bei der elektronischen Steuerungseinheit 19 sind weiterhin das Medikament und das Inhalationsvolumen über eine Eingabeeinheit 25, beispielsweise eine Tastatur oder einzelne Eingabetasten, einstellbar.

Ferner weist die erfindungsgemäße Inhalationsvorrichtung einen SmartCard-Leser 21 auf, über den auf einer SmartCard 22 gespeicherte individuelle Inhalationsparameter wie Inhalationsfluss und Inhalationsvolumen eingelesen werden können. Alternativ dazu werden die individuellen Parameter im Speicher 19c abgelegt.

Ein Netzteil 23 (beispielsweise 110 - 240 V) versorgt die Inhalationsvorrichtung 10 mit Strom.

Die Inhalation wird in der Form durchgeführt, dass der Patient am Mundstück 11 saugt, wodurch der Drucksensor 14 anspricht und automatisch die Inhalation startet. Für die Dauer der Inhalationszeit wird der Vernebler 12 über die Pumpe 16 mit Druckluft versorgt, und aus dem Mundstück 11 tritt mit dem vorgewähltem bzw. vorgegebenen Fluss das gewünschte Medikament in Aerosolform aus. Nach Ablauf der Inhalationszeit bzw. Erreichen des Inhalationsvolumens wird die Luftzufuhr unterbrochen und der Patient kann nicht weiter inhalieren. Dies bedeutet, dass erfindungsgemäß die Steuereinheit die Luftpumpe nur dann ansteuert bzw. aktiviert, wenn der Beginn eines Inhalationsvorganges von dem Drucksensor 14 erkannt wird. Ist der Inhalationsvorgang abgeschlossen, wird die Luftpumpe 16 von der Steuerungseinheit 19 wieder deaktiviert.

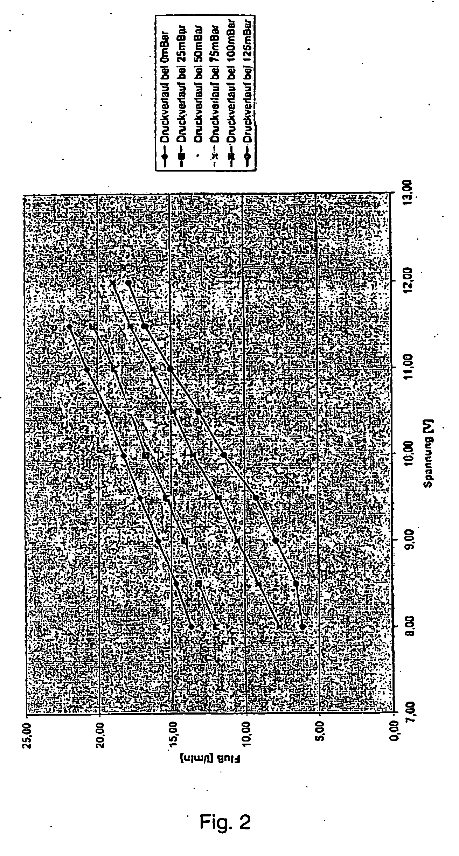

Unter Bezugnahme auf Fig. 2 wird nun ein bevorzugter Aspekt der vorliegenden Erfindung erläutert. Fig. 2 zeigt verschiedene Fluss-Spannung-Kennlinien für verschiedene vom Patienten während der Inhalation erzeugte Gegendrücke. Grundsätzlich ist Fig. 2 zu entnehmen, dass mit zunehmender Spannung, d.h. Steuerspannung der Pumpe 16, der bereitgestellte Fluss zunimmt. Für eine bestimmte Spannung, beispielsweise 10,0 V, nimmt der von der Pumpe bereitgestellte Fluss jedoch mit zunehmendem Gegendruck des Patienten ab. In Fig. 2 entspricht die oberste Kennlinie einem Gegendruck von 0 mbar, während die unterste Kennlinie einem Gegendruck von 125 mbar entspricht. Die dazwischen liegenden Kurven entsprechen Drücken von 25, 50, 75 und 100 mbar. Dies bedeutet, dass zur Korrektur des Inhalationsflusses bei variierendem Gegendruck die Pumpenspannung entsprechend variiert werden muß. Steigt beispielsweise der vom Patienten erzeugte Gegendruck an, sinkt der Fluss bei fest vorgegebener Spannung ab. Die Spannung muß deshalb erhöht werden, um dann auf der für diesen Gegendruck gültigen Kennlinie wieder den erforderlichen Inhalationsfluss bereitzustellen. Entsprechendes gilt umgekehrt für eine Abnahme des vom Patienten erzeugten Gegendruckes. Erfindungsgemäß sind diese Kennlinien in der Steuereinrichtung hinterlegt, so dass die Steuereinrichtung abhängig vom vom Patienten erzeugten Gegendruck die Pumpe entsprechend ansteuern und die Spannung nachregeln kann.

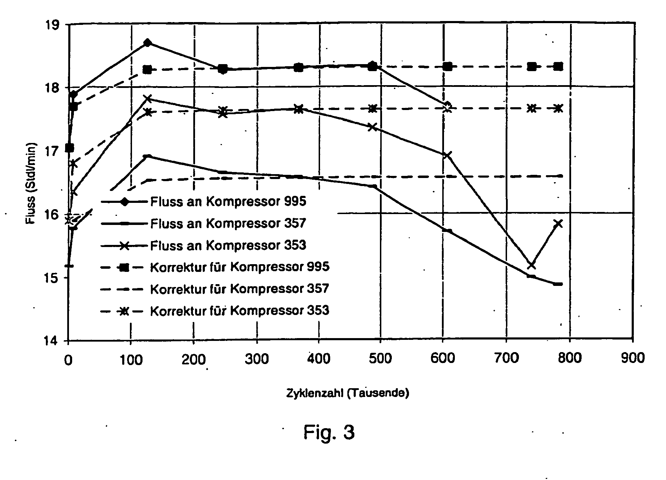

Fig. 3 erläutert eine weiter bevorzugte Korrekturmöglichkeit der erfindungsgemäßen Inhalationsvorrichtung. Fig. 3 zeigt Fluss-Zyklenzahl-Kennlinien von drei baugleichen Linien in Fig. 3 korrigierten Kennlinien entsprechen. Fig. 3 ist deutlich zu entnehmen, dass der von einer bestimmten Pumpe bereitgestellte Fluss während der ersten 100 bis etwa 120 Tausend Zyklen bis zu einem Maximum ansteigt, um dann jedoch allmählich abzufallen. Insbesondere nach 500000 Inhalationszyklen ist ein stärkerer Abfall zu erkennen. Für eine konkret in einer erfindungsgemäßen Inhalationsvorrichtung verwendete Luftpumpe wird vorab die Fluss-Spannung-Kennlinie messtechnisch ermittelt, da die Flusswerte der Kompressoren voneinander abweichen. Anschließend werden die Flusswerte anhand der Fluss-Zyklenzahl-Kennlinie mathematisch angenähert, so dass eine einfache Korrekturmöglichkeit für die Steuereinrichtung bereitgestellt wird.

Wie unterschiedlich die verschiedenen Fluss-Zyklenzahl-Kennlinien sein können, ist in der vorliegenden Tabelle veranschaulicht:

| Zyklenzahl (x) | Flussmittelwert (l/min) Pumpe 1 (0338000995) | Flussmittelwert (l/min) Pumpe 2 (0412000357) | Flussmittelwert (l/min) Pumpe 3 (0412000353) | Abweichung vom Anfangswert 1 (l/min) | Abweichung vom Anfangswert 2 (l/min) | Abweichung vom Anfangswert 3 (l/min) |
|---|---|---|---|---|---|---|
| 0 | 17,04 | 15,18 | 15,90 | 0,00 | 0,00 | 0,00 |
| 6000 | 17,89 | 15,78 | 16,36 | 0,84 | 0,60 | 0,46 |
| 125000 | 18,69 | 16,91 | 17,81 | 1,65 | 1,73 | 1,91 |
| 245500 | 18,27 | 16,65 | 17,57 | 1,23 | 1,47 | 1,67 |
| 366000 | 18,31 | 16,58 | 17,66 | 1,27 | 1,40 | 1,76 |
| 486000 | 18,33 | 16,43 | 17,35 | 1,29 | 1,24 | 1,45 |
| 606000 | 17,70 | 15,72 | 16,91 | 0,65 | 0,53 | 1,01 |
| 738500 | aus | 14,98 | 15,17 | aus | -0,20 | -0,74 |
| 781000 | aus | 14,86 | 15,83 | aus | -0,32 | -0,07 |

Für diese Messungen wurde eine Pumpenspannung von 11 V verwendet. Innerhalb eines Zyklusses waren die Pumpen 5s ein und 5s aus.

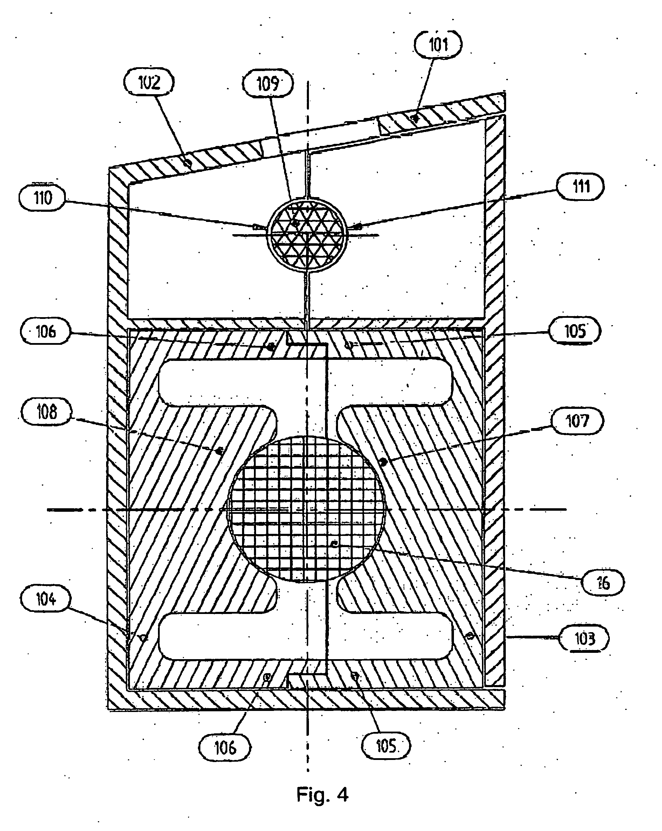

Fig. 4 zeigt eine bevorzugte Form der Lagerung der Luftpumpe 16 (Kompressor) in der Inhalationsvorrichtung. Gemäß dieser bevorzugten Lagerung weist das zweiteilige Gehäuse 101, 102 der Inhalationsvorrichtung an den jeweiligen Innenseiten schalenförmige Halterungen 103, 104 auf, die im zusammengebauten Zustand der Gehäuseteile die Luftpumpe 16 halten bzw. lagern. Vorzugsweise bestehen die schalenförmigen Halterungen 103, 104 aus einem Dämpfungsmaterial, z.B. einem entsprechenden ausgehärteten Dämpfungsschaum, so dass die Luftpumpe direkt von dem Dämpfungsmaterial gelagert wird. Dies verringert Montagekosten, da keine separaten Halterungen vorgesehen werden müssen, die die Luftpumpe zur Lagerung mit dem Gehäuse verbinden. Ferner ermöglicht dies einen leichten Austausch des Kompressors. Es müssen lediglich die beiden Gehäusehälften auseinander genommen werden und der Kompressor ist dann unmittelbar zugänglich und ohne weiteres entnehmbar; es müssen nicht erst etwaige Halterungen gelöst werden. Somit dient die Schallschutzeinrichtung gleichzeitig als Lagerung.

Die beiden schalenförmigen Halterungen sind vorzugsweise im jeweiligen Randbereich 105, 106 so ausgebildet, dass im zusammengebauten bzw. montierten Zustand die Kanten überlappen, um eine bessere Abdichtung zu erzielen.

Ferner weisen die schalenförmigen Halterungen wie in Fig. 4 gezeigt vorzugsweise entsprechende Projektionen 107, 108 auf, die in das Gehäuseinnere ragen, um den Kompressor zu lagern. Die Projektionen 107, 108 sind dazu in ihrer äußeren Form der Form des Kompressors angepasst.

Bezugszeichen 109 bezeichnet einen Stecker, der in den beiden halbkreisförmigen Gehäuseaussparungen 110 und 111 gehalten wird bzw. befestigt ist.

## Patentansprüche

1. Inhalationsvorrichtung (10) mit einer Steuereinrichtung (19) einem Vernebler (12) und einem dem Vernebler (12) zugeordneten Mundstück (11), einem mit der Steuereinrichtung (19) verbundenen Kompressor (16), in Leitungsverbindung mit den Mundstück (11), wobei der Kompressor (16) einen Konstanten Inhalationsfluss über die Leitungsverbindung an das Mundstück (11) liefert, wobei die Steuereinrichtung (19) den Kompressor derart über Spannung oder über Pulsweitenmodulation so direkt ansteuert, dass er der Inhalationsfluss gemäß einem vorgegebenen zeitlichen Verlauf an das znden Kompressor (16) drosselventilfrei angeschlossene Mundstück (11) liefert, wobei die Steuereinrichtung (19) über eine Verneblerelektronik (24) mit dem Vernebler (12) Verbunden ist.

2. Inhalationsvorrichtung nach Anspruch 1, wobei die Steuerung des Kompressors (16) durch die Steuereinrichtung (19) abhängig von patienten-individuellen Lungenfunktionsparametern erfolgt.

3. Inhalationsvorrichtung nach Anspruch 2, wobei die patienten-individuellen Lungenfunktionsparameter Inhalationsfluss und/oder Inhalationsvolumen sind.

4. Inhalationsvorrichtung nach Anspruch 2 oder 3, wobei die Steuereinrichtung (19) anhand der patienten-individuellen Lungenfunktionsparameter diejenige Steuerspannung für den Kompressor (16) ermittelt, die zur Erzielung des für den Patienten individuell vorgegebenen konstanten Inhalationsflusses $Q_{Soll}$ erforderlich ist

5. Inhalationsvorrichtung nach einem der Ansprüche 1 bis 4, wobei die Steuereinrichtung (19) eine Steuereinheit (19a) für den Antrieb des Kompressors (16), eine Verarbeitungseinheit (19b) und einen Speicher (19c) aufweist.

6. Inhalationsvorrichtung nach Anspruch 5, wobei in den Speicher (19c) Daten ablegbar sind, die die patienten-individuellen Lungenfunktionsparameter darstellen.

7. Inhalationsvorrichtung nach einem der Ansprüche 1 bis 6, ferner mit einer Leseeinrichtung (21) zum Lesen externer Datenträger (22).

8. Inhalationsvorrichtung nach Anspruch 7, wobei die Leseeinrichtung (21) ein SmartCard-Leser und der externe Datenträger (22) eine SmartCard ist, auf der Daten gespeichert sind, die die patienten-individuellen Lungenfunktionsparameter darstellen.

9. Inhalationsvorrichtung nach einem der Ansprüche 1 bis 8, ferner mit einer Anzeigeeinheit (20) und einer Eingabeeinheit (25).

10. Inhalationsvorrichtung nach einem der Ansprüche 1 bis 9, ferner mit einem Überdruckventil (13) abstromseitig vom Kompressor (16).

11. Inhalationsvorrichtung nach einem der Ansprüche 1 bis 10 ferner mit einem Drucksensor (14) abstromseitig vom Kompressor (16).

12. Inhalationsvorrichtung nach einem der Ansprüche 1 bis 11, mit einem Drucksensor (14) zum Erfassen des Beginns eines Inhalationsvorganges, wobei das zu inhalierende Medikament und/oder der Inhalationsfluss oder das Inhalationsvolumen vom Nutzer individuell einstellbar ist, und wobei bei Erfassung der ersten Inhalationsvorganges durch den Drucksensor (14) der/die individuell eingestellten Parameter fest eingestellt werden und der Kompressor (16) aktiviert wird.

13. Inhalationsvorrichtung nach Anspruch 12, wobei nach einer ersten erfolgten Inhalation oder einem Inhalationsabbruch der Nutzer wahlweise mit dem/den zuvor eingestellten Parameter(n) fortfahren oder eine Neueinstellung des/der Parameter vornehmen kann.

**Claims**

1. An inhalation device (10) comprising a control (19), a nebulizer (12) and a mouthpiece (11) associated with the nebulizer (12), an air pump (16) connected to the control (19), in operative connection with the mouthpiece (11), wherein the air pump (16) supplies a constant inhalation flow via the operative connection to the mouthpiece (11), wherein the control (19) directly controls the air pump such by voltage or pulse-width modulation that it supplies the inhalation flow according to a predetermined time course to the mouthpiece (11) connected to the air pump (16) without a throttle valve, wherein the control (19) is connected with the nebulizer (12) via nebulizer electronics (24).

2. The inhalation device according to claim 1, wherein the control of the air pump (16) is carried out via the control (19) depending on patient-individual lung function parameters.

3. The inhalation device according to claim 2, wherein the patient-individual lung function parameters are inhalation flow and/or inhalation volume.

4. The inhalation device according to claim 2 or 3, wherein the control (19) evaluates, on the basis of the patient-individual lung function parameters, the control voltage for the air pump (16) which is necessary for obtaining the constant inhalation flow $Q_{target}$ individually determined for the patient.

5. The inhalation device according to any one of claims 1 to 4, wherein the control (19) comprises a control unit (19a) for the drive of the air pump (16), a processing unit (19b) and a memory (19c).

6. The inhalation device according to claim 5, wherein data can be stored in the memory (19c) representing patient-individual lung function parameters.

7. The inhalation device according to any one of claims 1 to 6, further comprising a reader (21) to read external data carriers (22).

8. The inhalation device according to claim 7, wherein the reader (21) is a SmartCard reader and the external data carrier (22) is a SmartCard on which data are stored representing the patient-individual lung function parameters.

9. The inhalation device according to any one of claims 1 to 8, further comprising a display unit (20) and an input unit (25).

10. The inhalation device according to any one of claims 1 to 9, further comprising a safety valve (13) arranged downstream from the air pump (16).

11. The inhalation device according to any one of claims 1 to 10, further comprising a pressure sensor (14) arranged downstream from the air pump (16).

12. The inhalation device according to any one of claims 1 to 11, comprising a pressure sensor (14) for recognising the start of an inhalation process, wherein the pharmaceutical to be inhaled and/or the inhalation flow or the inhalation volume can be individually set by the user, and wherein upon recognition of the first inhalation process by the pressure sensor (14), the individual parameter(s) is/are set and the air pump (16) is activated.

13. The inhalation device according to claim 12, wherein after a first inhalation or an inhalation stop, the user may optionally continue with the parameter(s) set before or perform a reset of the parameter(s).

**Revendications**

1. Dispositif d'inhalation (10) comprenant un système de commande (19), un nébulisateur (12) et un embout buccal (11) associé audit nébulisateur (12), un compresseur (16) relié au système de commande (19) et en liaison par conduit avec l'embout buccal (11), ledit compresseur (16) délivrant un flux d'inhalation constant audit embout buccal (11) par l'intermédiaire de la liaison par conduit, le système de commande (19) activant directement ledit compresseur, par tension ou par modulation de largeur d'impulsions, de façon telle qu'il délivre le flux d'inhalation, conformément à une allure temporelle préétablie, à l'embout buccal (11) raccordé audit compresseur (16) avec absence de vanne d'étranglement, ledit système de commande (19) étant connecté au nébulisateur (12) par l'intermédiaire d'une électronique de nébulisation (24).

2. Dispositif d'inhalation selon la revendication 1, dans lequel la commande du compresseur (16), sous l'action du système de commande (19), s'opère d'une manière dépendant de paramètres individuels de la fonction pulmonaire d'un patient.

3. Dispositif d'inhalation selon la revendication 2, dans lequel les paramètres individuels de la fonction pulmonaire d'un patient sont un flux d'inhalation et/ou un volume d'inhalation.

4. Dispositif d'inhalation selon la revendication 2 ou 3, dans lequel, à l'appui des paramètres individuels de la fonction pulmonaire d'un patient, le système de commande (19) détermine la tension de commande dédiée au compresseur (16) et nécessaire à l'obtention du flux d'inhalation constant $Q_{soll}$ préétabli individuellement pour le patient.

5. Dispositif d'inhalation selon l'une des revendications 1 à 4, dans lequel le système de commande (19) comporte une unité de commande (19a) dévolue à l'entraînement du compresseur (16), une unité de traitement (19b) et une mémoire (19c).

6. Dispositif d'inhalation selon la revendication 5, dans lequel des données, représentant les paramètres individuels de la fonction pulmonaire d'un patient, peuvent être déposées dans la mémoire (19c).

7. Dispositif d'inhalation selon l'une des revendications 1 à 6, par ailleurs équipé d'un système de lecture (21) conçu pour lire des supports de données extérieurs (22).

8. Dispositif d'inhalation selon la revendication 7, dans lequel le système de lecture (21) est un lecteur de cartes à puces, et le support de données extérieur (22) est une carte à puce sur laquelle sont mémorisées des données représentant les paramètres individuels de la fonction pulmonaire d'un patient.

9. Dispositif d'inhalation selon l'une des revendications 1 à 8, par ailleurs équipé d'une unité d'affichage (20) et d'une unité d'entrée (25).

10. Dispositif d'inhalation selon l'une des revendications 1 à 9, par ailleurs équipé d'une vanne de surpression (13) en aval du compresseur (16).

11. Dispositif d'inhalation selon l'une des revendications 1 à 10, par ailleurs équipé d'un capteur de pression (14) en aval du compresseur (16).

12. Dispositif d'inhalation selon l'une des revendications 1 à 11, équipé d'un capteur de pression (14) conçu pour détecter le début d'un processus d'inhalation, sachant que le médicament à inhaler et/ou le flux d'inhalation et/ou le volume d'inhalation peu(ven)t être réglé(s) individuellement par l'utilisateur et sachant que, lors d'une détection du premier processus d'inhalation par ledit capteur de pression (14), le/les paramètre(s) réglé(s) individuellement est/sont consigné(s) et le compresseur (16) est activé.

13. Dispositif d'inhalation selon la revendication 12, dans lequel, à l'issue d'une première inhalation ou après une interruption d'inhalation, l'utilisateur peut sélectivement poursuivre avec le/les paramètre(s) préalablement réglé(s), ou procéder à un nouveau réglage du/des paramètre(s).

Fig. 1

Fig. 2

Zyklenzahl (Tausende)

Fig. 3

Legend within figure:
- Fluss an Kompressor 995
- Fluss an Kompressor 357
- Fluss an Kompressor 353
- Korrektur für Kompressor 995
- Korrektur für Kompressor 357
- Korrektur für Kompressor 353

Y-axis: Fluss (Std/min)

EP 1 700 614 B1

Fig. 4

13

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9852633 A
- EP 1258264 A
- WO 0058022 A
- DE 19939417 A
- DE 10022795 A
- DE 202004004809 U1